(19) Europäisches Patentamt
European Patent Office
Office européen des brevets

(11) Veröffentlichungsnummer: **0 051 784**
**A1**

(12) # EUROPÄISCHE PATENTANMELDUNG

(21) Anmeldenummer: **81108890.5**

(22) Anmeldetag: **24.10.81**

(51) Int. Cl.³: **C 07 D 231/12**, A 01 N 43/56

(30) Priorität: **07.11.80 DE 3041998**

(43) Veröffentlichungstag der Anmeldung: **19.05.82 Patentblatt 82/20**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Brandes, Wilhelm, Dr., Eichendorffstrasse 3, D-5653 Leichlingen 1 (DE)**
Erfinder: **Daum, Werner, Dr., Bärenstrasse 18, D-4150 Krefeld (DE)**

(54) **Pyrazolsubstituierte Oximino-cyan-acetamid-Derivate, Verfahren zu ihrer Herstellung sowie ihre Verwendung als Fungizide.**

(57) Die Erfindung betrifft pyrazolsubstituierte Oximinocyan-acetamid-Derivate der Formel I, mehrere Verfahren zu ihrer Herstellung sowie ihre Verwendung als Fungizide,

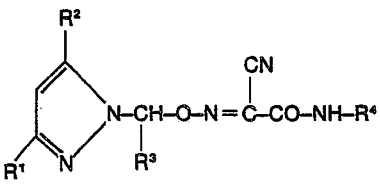
(I)

in welcher

$R^1$ und $R^2$ Wasserstoff und Alkyl

$R^3$ Wasserstoff, Alkyl Halogenalkyl

$R^4$ Wasserstoff und CO–NH–$R^5$ wobei

$R^5$ Wasserstoff und Alkyl welche durch Cyano-, Hydroxycarbonyl-, Amino-Carbonyl- und Alkoxycarbonyl-Gruppen mit bis zu 4 Kohlenstoffatomen im Alkylteil substituiert sein kann.

Die Wirkstoffe sind für den Gebrauch als Pflanzenschutzmittel geeignet und können zur Bekämpfung von Oomyceten eingesetzt werden.

EP 0 051 784 A1

0051784

BAYER AKTIENGESELLSCHAFT
Zentralbereich
Patente, Marken und Lizenzen

5090 Leverkusen, Bayerwerk
Slr/kl-c
Ib

# Pyrazolsubstituierte Oximino-cyan-acetamid-Derivate, Verfahren zu ihrer Herstellung sowie ihre Verwendung als Fungizide

Die vorliegende Erfindung betrifft neue pyrazolsubstituierte Oximino-cyan-acetamid-Derivate, mehrere Verfahren zu ihrer Herstellung sowie ihre Verwendung als Fungizide.

Wie bereits lange bekannt ist, werden als Fungizide in der Landwirtschaft und im Gartenbau insbesondere das Zink-ethylen-1,2-bis-dithiocarbamidat und das N-Trichlormethylthio-tetrahydrophthalimid verwendet; die genannten Verbindungen besitzen unter den Handelsprodukten eine große Bedeutung (vgl. R. Wegler, "Chemie der Pflanzenschutz- und Schädlingsbekämpfungsmittel", Band 2, Seiten 65 und 108, Berlin/Heidelberg/New York (1970)). Die Wirkung bei niedrigen Aufwandkonzentrationen ist jedoch nicht immer befriedigend. Auch sind diese Fungizide nicht curativ einsetzbar.

Weiterhin aus einer Reihe von Patentschriften bekannt ist die fungizide Wirkung von einigen Isonitroso-cyan-acetamid-Derivaten (vgl. hierzu z.B. die Deutsche Offenlegungsschrift 2 312 956 und die US-Patentschriften 3 919 284, 3 957 847 und 4 188 401). Auch hier ist die Wirksamkeit bei niedrigen Aufwandmengen nicht zuverlässig und bei normalen Konzentrationen werden oft Pflanzenschäden gesehen.

Es wurden nun als neue Stoffe die pyrazolsubstituierten Oximino-cyan-acetamid-Derivate der allgemeinen Formel

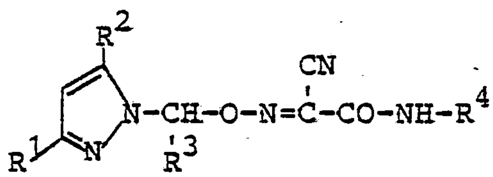

(I)

in welcher

$R^1$ und $R^2$ für Wasserstoff und Alkyl mit bis zu 3 Kohlenstoffatomen stehen,

$R^3$ für Wasserstoff, Alkyl mit bis zu 6 Kohlenstoffatomen und Halogenalkyl mit bis zu 2 Kohlenstoffatomen und bis zu 5 Halogenatomen steht,

$R^4$ für Wasserstoff und die Gruppe $CO-NH-R^5$ steht, wobei

$R^5$ für Wasserstoff und Alkyl mit bis zu 8 Kohlenstoffatomen steht, wobei letzteres durch Cyano-, Hydroxycarbonyl-, Aminocarbonyl- und Alkoxycarbonyl-Gruppen mit bis zu 4 Kohlenstoffatomen im Alkylteil substituiert sein kann,

gefunden.

Die erfindungsgemäßen Stoffe der allgemeinen Formel (I) können als Oximderivate in 2 verschiedenen geometrischen Strukturen vorliegen:

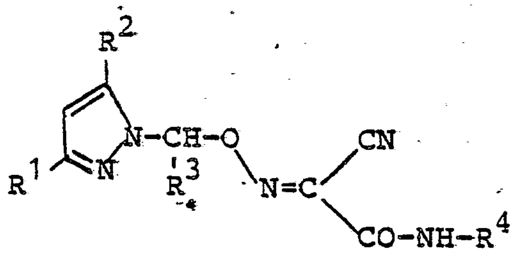

E-Derivat

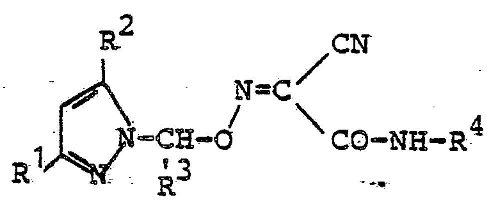

Z-Derivat

Im folgenden wird auf die Angabe der räumlichen Struktur verzichtet; für die Zwecke der vorliegenden Anmeldung sollen die angegebenen Formeln in jedem Fall auch die entsprechende Formel gemäß der geometrischen Struktur E oder Z mitumfassen.

Weiterhin wurde gefunden, daß man die pyrazolsubstituierten Oximino-cyan-acetamid-Derivate der Formel (I) erhält, wenn man

a) ein N-Alkylpyrazol der Formel

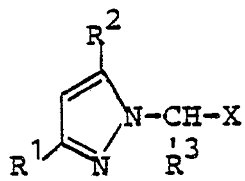

(II)

in welcher

$R^1$, $R^2$ und $R^3$ die oben angegebenen Bedeutungen besitzen und

X für eine Abgangsgruppe wie Chlor, Brom, Jod oder eine Sulfonyloxygruppe steht,

mit einem 2-Oximino-2-cyan-acetamid-Derivat der Formel

$$HO-N=\overset{\displaystyle CN}{\overset{|}{C}}-CO-NH-R^4 \qquad (III)$$

in welcher

$R^4$ die obengenante Bedeutung besitzt,

umsetzt; ferner daß man

b) diejenigen pyrazolsubstituierten Oximino-cyan-acetamid-Derivate der Formel (I), bei denen $R^4$ für Wasserstoff und die Gruppe $CO-NH_2$ steht, auch dann erhält, wenn man ein N-Alkylpyrazol der Formel (II) mit einem Alkali- oder Erdalkalisalz von 2-Oximino-2-cyan-acetamid bzw. von N-(2-Oximino-2-cyan-acetyl)-harnstoff (somit mit solchen Verbindungen der allgemeinen Formel (III), in welchen $R^4$ für Wasserstoff bzw. die Gruppe $CO-NH_2$ steht) umsetzt; ferner daß man

c) diejenigen pyrazolsubstituierten Oximino-cyan-acetamid-Derivate der Formel (I), bei denen $R^4$ für die Gruppe $CO-NH-R^5$ steht, wobei $R^5$ für die obengenannten Bedeutungen unter Ausschluß von Wasserstoff und Hydroxycarbonylalkyl steht, auch dann erhält, wenn man gemäß den Verfahren a) oder b) hergestellte erfindungsgemäße Verbindungen der Formel

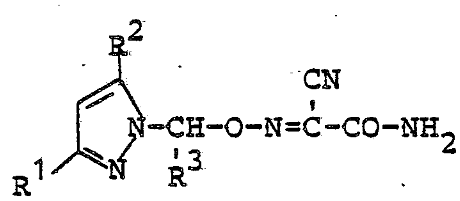

(IV)

in welcher

$R^1$, $R^2$ und $R^3$ die obengenannten Bedeutungen besitzen

(die Formel (IV) ist in dem Fall mit Formel (I) identisch, wenn der Substituent $R^4$ in Formel (I) für Wasserstoff steht),

in Gegenwart einer starken Base mit einem Isocyanat der Formel

$$OCN-R^5 \qquad (V)$$

in welcher

$R^5$ die obengenannten Bedeutungen mit Ausnahme von Wasserstoff und Hydroxycarbonylalkyl besitzt,

umsetzt; und schließlich gemäß einer weiteren Verfahrensvariante

d) zur Herstellung solcher Verbindungen der Formel (I), bei denen $R^4$ für $CO-NH-R^5$ steht und $R^5$ dabei die Bedeutung Alkyl-carbonyloxyalkyl annimmt, eine nach Verfahren a) synthetisierte Carbonsäure der Formel

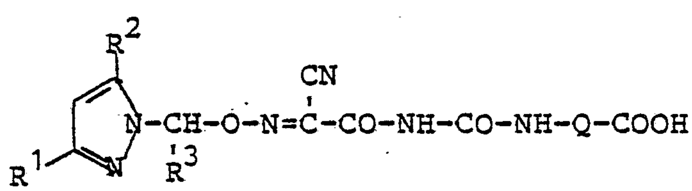

(VI)

in welcher

$R^1$, $R^2$ und $R^3$ die obengenannten Bedeutungen besitzen und

Q für gerades oder verzweigtes Alkylen mit 1 bis 5 Kohlenstoffatomen steht

(die Verbindungen der Formel (VI) sind mit den Verbindungen der Formel (I) in dem Falle identisch, wenn der Substituent $R^4$ in Formel (I) für die Gruppe $CO-NH-R^5$ steht und dabei $R^5$ die Bedeutung Hydroxycarbonylalkyl besitzt),

in Form ihres Alkali-, Erdalkali- oder Ammoniumsalzes mit einem Alkylierungsmittel der Formel

$$Y-R^6 \qquad (VII)$$

in welcher

$R^6$ für Alkyl mit 1 bis 3 Kohlenstoffatomen steht und

Y für eine Abgangsgruppe wie Chlor, Brom, Jod, Alkoxysulfonyloxy, Alkylsulfonyloxy oder Arylsulfonyloxy steht,

umsetzt.

Die neuen pyrazolsubstituierten Oximino-cyan-acetamid-Derivate weisen starke fungizide Eigenschaften auf. Sie sind protektiv, curativ und sogar eradikativ anwendbar, außerdem haben sie systemische und/oder locosystemische Eigenschaften. Überraschenderweise zeigen sie eine bessere Pflanzenverträglichkiet als die nach dem Stand der Technik bekannten Isonitrosocyanacetamid-Derivate. Gegenüber den Dithiocarbamidaten und dem N-Trichlormethylthio-tetrahydrophthalimid besitzen sie den Vorteil curativer und eradikativer Wirkung.

Die erfindungsgemäßen Verbindungen stellen schon wegen der vielen Möglichkeiten ihrer überlegenen biologischen Anwendung eine wertvolle Bereicherung der Technik dar. Ein weiterer wesentlicher Gesichtspunkt dieser Erfindung ist, daß neue Wirkstoffe mit für die Praxis wertvollen Eigenschaften zu einer Zeit zur Verfügung gestellt werden, da durch Resistenzerscheinungen ältere Wirkstoffe für den Markt ausfallen.

Es besteht daher heute und in absehbarer Zeit ein ausgesprochener Bedarf nach neuen Fungiziden, die dann allein oder ggf in Kombination mit anderen Fungiziden mit nicht gleichem Wirkungsmechanismus oder in alternierenden Bekämpfungsprogrammen mit diesen angewendet werden können.

Von den erfindungsgemäßen pyrazolsubstituierten Oximino-cyan-acetamid-Derivaten der Formel (I) sind bevorzugt diejenigen, in denen $R^1$ und $R^2$ für Wasserstoff und Methyl stehen, $R^3$ für Wasserstoff und Alkyl mit 1 bis 4 Kohlenstoffatomen steht, $R^4$ für Wasserstoff und die Gruppe $CO-NH-R^5$ steht, wobei $R^5$ für Wasserstoff und gegebenenfalls cyansubstituiertes Alkyl mit 1 bis 5 Kohlenstoffatomen steht.

Besonders bevorzugt sind diejenigen Verbindungen der Formel (I), in denen $R^1$ und $R^2$ für Wasserstoff stehen, $R^3$ für Wasserstoff, Methyl, Ethyl, Isopropyl und sek.-Butyl und $R^4$ für die obengenannten Bedeutungen steht, wobei $R^5$ für Wasserstoff, Methyl, Ethyl, n-Propyl und Isopropyl steht.

Verwendet man zur Herstellung der erfindungsgemäßen Verbindungen der allgemeinen Formel (I) beispielsweise gemäß Verfahrensvariante a) 1-Chlormethyl-pyrazolhydrochlorid und 1-(2-Oximino-2-cyan-acetyl)-3-ethyl-harnstoff als Ausgangsstoffe, sowie Ethyl-diisopropylamin als Protonenakzeptor, so kann der Reaktionsverlauf durch das folgende Formelschema wiedergegeben werden:

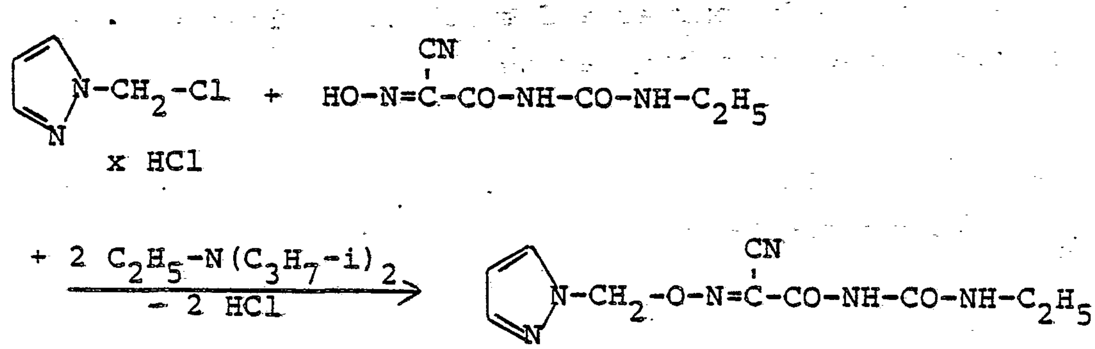


Verwendet man beispielsweise gemäß Verfahrensvariante b) 1-Chlor-1-pyrazolyl-2-methyl-propan und das Kaliumsalz des 2-Oximino-2-cyan-acetamids als Ausgangsstoffe,

so kann der Reaktionsverlauf durch das folgende Formelschema wiedergegeben werden:

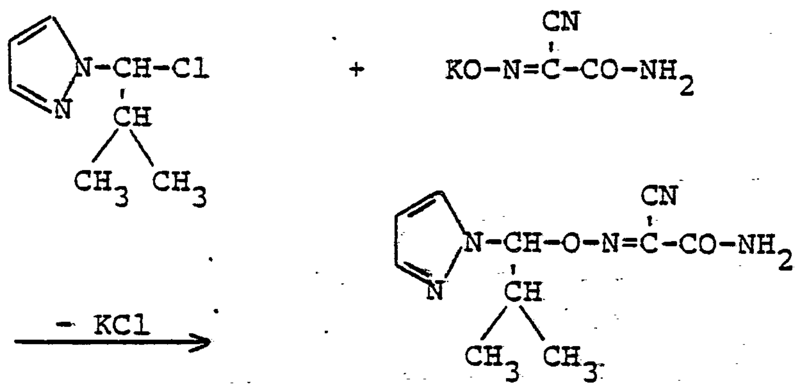


Verwendet man beispielsweise gemäß Verfahrensvariante c) 2-(Pyrazolylmethyl-oximino)-2-cyan-acetamid, Natriumhydrid und n-Butylisocyanat als Ausgangsstoffe, so kann der Reaktionsverlauf durch das folgende Formelschema wiedergegeben werden:

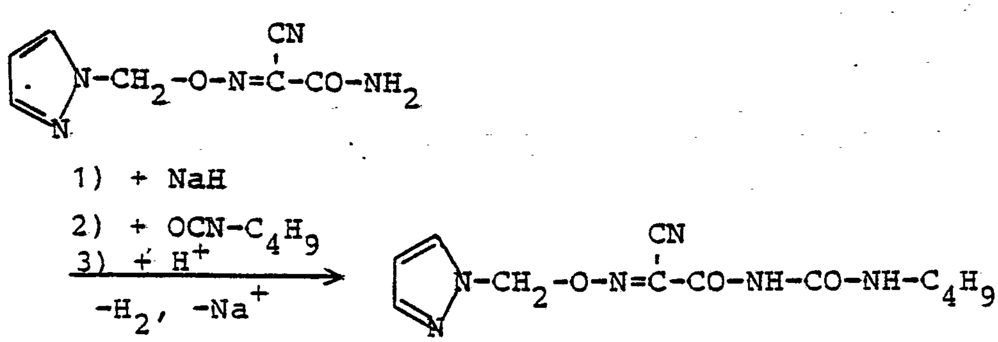

Verwendet man beispielsweise gemäß Verfahrensvariante d) 1-[2-(Pyrazolylmethyl-oximino)-2-cyan-acetyl]-3-[5-(hydroxycarbonyl)-pentyl]-harnstoff in Form seines Natriumsalzes und Methyljodid als Ausgangsstoffe, so kann der Reaktionsverlauf durch das folgende Formelschema wiedergegeben werden:

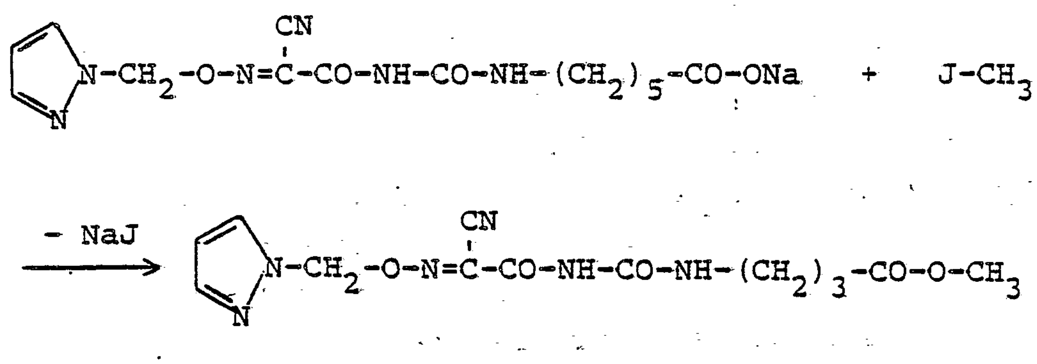


Die nach Verfahrensvariante a) oder b) als Ausgangssubstanzen benötigten N-Alkylpyrazole der Formel (II) sind teilweise bekannt (J. Chem. Soc. 1960, 5272-3; DE-OS 2 835 158; Chem. Ber. 85, 820 (1952)). Man kann sie z.B. dadurch erhalten, daß man einen Aldehyd mit einem Pyrazol, gegebenenfalls in einem inerten Lösungsmittel, zur Reaktion bringt und anschließend mit z.B. Thionylhalogenid umsetzt, wobei in diesem Falle die Hydrohalogenide der N-Alkylpyrazole anfallen.

Zu nennen sind hier beispielsweise die folgenden Verbindungen: 1-Chlormethyl-, 1-(1-Chlor-ethyl)-, 1-(1,2-Dichlorethyl)-, 1-(1-Chlor-2-methyl-propyl)-,

1-(1-Chlormethyl-propyl)-pyrazol-, -3- bzw. -5-methylpyrazol- und -3,5-dimethylpyrazol-hydrochlorid sowie 1-(1-Bromethyl)-pyrazol-hydrobromid.

Weiterhin werden für die erfindungsgemäßen Umsetzungen nach Verfahrensvariante a) oder b) die 2-Oximino-2-cyan-acetamid-Derivate der allgemeinen Formel (III) benötigt. Die Verbindungen sind teilweise bekannt (vgl. Chem. Ber. 42, 738 - 741 (1909); 54, 1334 (1921); US-PS 4 188 401). Man erhält sie, wenn man z.B. Ethylisocyanat in einer ersten Stufe mit Ammoniak zu N-Ethyl-harnstoff, in einer zweiten Stufe diesen mit Cyanessigsäure in Gegenwart von Acetanhydrid umsetzt, und den so erhaltenen 1-Ethyl-3-(2-cyanacetyl)-harnstoff mit salpetriger Säure oximiert.

Die für die Verfahrensvariante c) erforderlichen Isocyanate sind allgemein bekannte Verbindungen; sie werden in üblicher Weise erhalten, wenn man z.B. primäre Amine mit Phosgen umsetzt.

Zu nennen sind hier: Methyl-, Ethyl-, Propyl-, Isopropyl-, Butyl-, sek.-Butyl-, ω-Cyanethyl-, 1-Cyan-1-methyl-ethyl-, ω-Cyan-propyl-, ω-Cyanpentyl-, Methoxycarbonyl-methyl-, Ethoxycarbonyl-methyl-, Propoxycarbonyl-ethyl-, 1-Methoxycarbonyl-1-methyl-ethyl-, 1-Ethoxycarbonyl-1-methyl-ethyl-, Ethoxycarbonyl-1-ethyl-ethyl-, Methoxycarbonyl-1-ethyl-ethyl-, Methoxycarbonyl-propyl-, Methoxycarbonylpentyl-, Isopropoxycarbonylpentyl-isocyanat.

Als Verdünnungsmittel kommen für das Verfahren gemäß Variante a) alle gegenüber den Reaktionspartnern inerten organischen Lösungsmittel in Frage; vorzugsweise werden polare Lösungsmittel verwendet. Zu nennen sind hier beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran.

Die Reaktionen können auch in Mischungen aus Wasser und einem wassermischbaren organischen Lösungsmittel durchgeführt werden oder in heterogenen Systemen, bestehend aus Wasser und einem mit Wasser nicht mischbaren oder nur teilweise mischbaren Lösungsmittel.

Als Säurebinder für die Umsetzung nach Verfahren a) werden organische Basen verwendet, vorzugsweise terriäre Amine. Zu nennen sind hier: Chinolin, Dimethylbenzylamin, Dimethylanilin, Ethyldicyclohexylamin, Ethyldiisopropylamin, Picolin, Pyridin und Triethylamin.

Die Reaktionstemperaturen und die Reaktionsdauer beim Verfahren a) werden durch die Aktivität der Ausgangsprodukte bestimmt. Im allgemeinen arbeitet man etwa zwischen -50 und +80°C, vorzugsweise zwischen -30 und +40°C. Bei Verwendung oder Mitverwendung von Wasser

als Verdünnungsmittel arbeitet man im Temperaturbereich zwischen dem Erstarrungspunkt der wäßrigen Lösung und etwa +60°C, vorzugsweise bei 0 bis +40°C.

Zur Durchführung des erfindungsgemäßen Verfahrens nach Variante a) wird das 2-Oximino-2-cyan-acetamid-Derivat der Formel (III), in den oben erwähnten Verdünnungsmitteln dispergiert oder gelöst, vorgelegt und mit einer molaren Menge eines tertiären Amins versetzt, wobei Salzbildung eintreten kann. Soweit die Umsetzung mit einem Salz der reaktiven N-Alkyl-pyrazol-Verbindung der Formel (II) erfolgen soll, wird man entweder vor Zugabe der Pyrazol-Verbindung oder aber gleichzeitig mit dieser nochmals eine molare Menge eines tertiären Amins eintragen. Die reaktive Pyrazol-Verbindung wird man vorzugsweise in flüssiger Form oder als Lösung in das Reaktionsgefäß einbringen. Die Reaktionsmischung sollte am Ende der Umsetzung alkalisch reagieren, danach aber bald schwach angesäuert werden.

Nach einer besonderen Verfahrensweise setzt man den Mischungen vor Beginn der Reaktion eine kleine Menge eines Jodids zu, wenn man nicht gerade eine Verbindung der Formel II mit Jod als Abgangsgruppe einsetzt. Hierdurch wird die Reaktionsgeschwindigkeit erhöht.

Als Verdünnungsmittel können beim Verfahren b) alle in Relation zu den Reaktionspartnern inerten Lösungsmittel verwendet werden. Zu nennen sind hier polare Lösungsmittel, wie z.B. Acetonitril, Dimethylformamid, Dimethylacetamid, Dimethylsulfoxid, oder, wenn in Suspension gearbeitet werden soll, aromatische Kohlenwasserstoffe wie z.B. Chlorbenzol und Toluol.

Die Reaktionstemperaturen beim Verfahren b) können ebenfalls in einem größeren Bereich variiert werden. Im allgemeinen arbeitet man zwischen -20 und +60°C, vorzugsweise zwischen -10 und +30°C.

Zur Durchführung des erfindungsgemäßen Verfahrens nach Variante b) wird ein Alkali- oder Erdalkalisalz des 2-Oximino-2-cyan-acetamid-Derivates in einem inerten Lösungsmittel vorgelegt, oder man erzeugt das Salz, indem man zu einer Mischung aus dem 2-Oximino-2-cyan-acetamid-Derivat und einem hochsiedenden Lösungsmittel Alkalilauge, Alkoholate oder ein Erdalkalihydroxid gibt und dann vorsichtig entwässert bzw. den Alkohol abdestilliert.

Zur Umsetzung der Salze der reaktiven N-Alkylpyrazole ist es zweckmäßig, noch eine molare Menge eines tertiären Amins zusätzlich zu verwenden.

Als Verdünnungsmittel kommen für das Verfahren gemäß Variante c) indifferente wasserfreie Lösungsmittel in Frage, wie z.B. Ether, wie Diisopropylether, Dioxan oder Tetrahydrofuran.

Die Reaktionstemperaturen beim Verfahren c) können zwischen -20 und +80°C variiert werden, vorzugsweise arbeitet man zwischen +20 und 60°C.

Zur Durchführung des erfindungsgemäßen Verfahrens gemäß Variante c) wird ein 2-Cyan-2-pyrazol-1-yl-methyloximino-acetamid-Derivat der Formel (IV) in einem oben näher bezeichneten indifferenten Lösungsmittel mit Natriumhydrid oder Kalium-tert.-butanolat in das entsprechende Alkalisalz überführt und anschließend mit dem Isocyanat der Formel (V) in dem angegebenen Temperaturbereich umgesetzt. Nach beendeter Reaktion wird in der Kälte mit einer organischen Carbonsäure schwach angesäuert.

Als Verdünnungsmittel kommen für das Verfahren gemäß Variante d) alle indifferenten Lösungsmittel in Frage. Vorzugsweise zu nennen sind Acetonitril, Dioxan, oder Ketone, wie z.B. Aceton und Diethylketon.

Die Reaktionstemperaturen beim Verfahren d) können zwischen -20 und +80°C variiert werden, vorzugsweise arbeitet man zwischen +20 und 60°C.

Nach Verfahren d) wird man das nach Verfahren a) gewonnene 1-(2-Cyan-2-pyrazol-1-yl-methyl-oximino)-3-(ω-carboxyalkyl)-harnstoff-derivat in den angegebenen Lösungsmitteln, nach erfolgter Salzbildung mit einem

Alkali- oder Erdalkaliion bzw. mit einem tert. Amin, mit einem Alkylierungsmittel umsetzen.

Je nach Arbeitsbedingungen fallen die erfindungsgemäßen Wirkstoffe kristallin aus oder sie bleiben im organischen Lösungsmittel gelöst und können dann nach Auswaschen der Lösung mit Wasser durch vorsichtiges Einengen der Lösung oder durch Zugabe wenig polarer organischer Lösungsmittel, wie Cyclohexan, Dibutylether oder Tetrachlorkohlenstoff, abgeschieden werden. Gegebenenfalls müssen mit Wasser mischbare polare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie auch durch Zugabe von Wasser ausgefällt werden. Soweit es die besonderen Bedingungen der Aufarbeitungsprozesse erlauben, werden die Lösungen der erfindungsgemäßen Wirkstoffe, bzw. die noch Lösungsmittel-feuchten Suspensionen der Wirkstoffe schwach sauer eingestellt. Einige Verbindungen sind auch gut wasserlöslich.

Die erfindungsgemäßen Verbindungen zersetzen sich zum Teil bei höherer Temperatur; in diesen Fällen können die Schmelzpunkte nur mit geringer Genauigkeit oder überhaupt nicht ermittelt werden. Das Vorliegen bestimmter Strukturelemente ist aus den NMR-Spektren

zu entnehmen. Auch zeigen die IR-Spektren charakteristische Absorptionsbanden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Kartoffel und Tomate (Phytophthora infestans), eingesetzt werden.

Sie zeigen eine hohe kurative und protektive Wirksamkeit. Daneben sind gute Wirkungen gegen Pyricularia und gegen Rostpilze festzustellen.

Die erfindungsgemäßen Wirkstoffe weisen nicht nur die guten Eigenschaften hervorragender Handelspräparate auf, sondern besitzen darüber hinaus noch erhebliche Vorteile. Diese liegen in erster Linie in der Fähigkeit der erfindungsgemäßen Stoffe, in die Pflanze einzudringen. Sie können aufgenommen werden von der Saatgutoberfläche, von den Wurzeln und auch von oberirdischen Pflanzenorganen nach äußerlichen Applikationen. Auch besitzen sie die vorteilhafte Fähigkeit, locosystemisch zur Wirkung zu kommen, d.h. eine Tiefenwirkung im Pflanzengewebe auszuüben und dabei pilzliche Krankheitserreger zu eliminieren, die bereits in das Gewebe der Wirtspflanze eingedrungen sind.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln.

also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-

Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Zusätzlich zu den obigen Formulierungsmöglichkeiten ist zu bemerken, daß die erfindungsgemäßen Stoffe zusammen mit Saccharose, Dextrose, Dextrinen, mit wasserfreiem Calciumsulfat oder Calciumsulfat-hemihydrat, sowie mit Carbonsäuren, wie z.B. Fumarsäure oder 4-Hydroxylbenzoesäure, oder auch mit schwach sauren Ionenaustauschern formuliert werden können.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

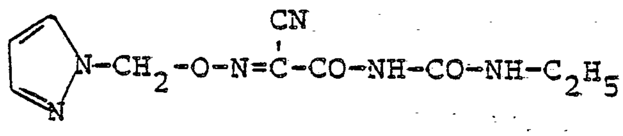

357 g 1-(2-Cyan-2-oximino-acetyl)-3-ethyl-harnstoff (1,94 Mol), suspendiert in 1,5 l trockenem Acetonitril, werden unter schwacher äußerer Kühlung mit 202 g Triethylamin (2 Mol) versetzt. Dann kühlt man auf etwa -30°C ab und läßt eine Lösung von 299 g N-Chlormethylpyrazol-hydrochlorid (1,95 Mol) in 2 l trockenem Acetonitril langsam zufließen. Gleichzeitig tropft man noch etwa 178 g Triethylamin (1,76 Mol) in das Reaktionsgefäß.

Nach Zugabe der Pyrazolkomponente wird die Reaktionsmischung 15 Minuten bei einem pH-Wert von 9 belassen. Gegebenenfalls ist noch etwas Triethylamin zuzufügen. Danach wird mit Essigsäure schwach angesäuert, das Kühlbad entfernt und die Hauptmenge des Acetonitrils im Vakuum abgedampft. Der Rückstand wird mit 800 g Eis und Wasser versetzt. Das kristalline Reaktionsprodukt wird abgetrennt und solange mit Wasser gewaschen, bis eine Probe kein Chlorid mehr enthält.

Ausbeute nach dem Trocknen bei 60°C/0,1 mbar: 379,6 g 1-[2-(Pyrazol-1-yl)-methoximino)-2-cyan-acetyl]-3-ethyl-harnstoff.

Das Produkt kann durch Lösen in Aceton und Ausfällen mit Wasser weiter gereinigt werden. Fp. 154°C.

Kontrolle durch Dünnschichtchromatographie auf Kieselgel, Fließmittel Choroform-Methanol-Toluol im Volumenteil-Verhältnis 1:1:1.

In entsprechender Weise werden die folgenden Verbindungen der allgemeinen Formel

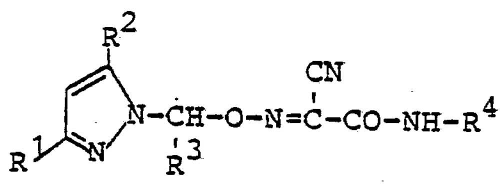

(I)

erhalten:

| Beispiel Nr. | $R^1$ | $R^2$ | $R^3$ | $R^4$ | Fp (°C) |
|---|---|---|---|---|---|
| 2 | H | H | H | H | 152,5 |
| 3 | H | H | H | $CO-NH-(CH_2)_5CN$ | 121 |
| 4 | H | H | H | $CO-NH_2$ | 210 |
| 5 | H | H | $CH_3$ | H | 172 |
| 6 | H | H | $CH_3$ | $CO-NH-C_2H_5$ | 149 |
| 7 | H | H | $CH(CH_3)_2$ | H | 151,5 |
| 8 | H | H | $CH(CH_3)_2$ | $CO-NH-CH_3$ | 167 |
| 9 | H | H | $CH(CH_3)_2$ | $CO-NH-C_2H_5$ | 122 |
| 10 | $CH_3$ | $CH_3$ | H | $CO-NH-C_2H_5$ | 163 |
| 11 | $CH_3$ | $CH_3$ | H | $CO-NH-(CH_2)_5-CN$ | 111,5 |
| 12 | $CH_3$ | $CH_3$ | H | $CO-NH_2$ | 202 |
| 13 | $CH_3$ | $CH_3$ | H | H | 142 |

Beispiel A

Phytophthora-Test (Tomaten) / Protektiv

| | | |
|---|---|---|
| Lösungsmittel | 4,7 Gewichtsteile | Aceton |
| Emulgator | 0,3 Gewichtsteile | Alkylarylpolyglykoläther |
| Wasser | 95 Gewichtsteile | |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70 % im Gewächshaus. Anschließend werden die Tomatenpflanzen mit einer wäßrigen Sporensuspension von Phytophthora infestans inokuliert. Die Pflanzen werden in eine Feuchtkammer mit einer 100 %igen Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Eine deutliche Überlegenheit in der Wirksamkeit gegenüber dem Stand der Technik zeigen in diesem Test z.B. die Verbindungen gemäß folgender Herstellungsbeispiele: 5 und 7.

Beispiel B

Phytophthora-Test (Tomaten) / Systemisch

| | | |
|---|---|---|
| Lösungsmittel: | 4,7 Gewichtsteile | Aceton |
| Dispergiermittel: | 0,3 Gewichtsteile | Alkyl-arylpolyglykolether |
| Wasser: | 95 Gewichtsteile | |

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden mit 10 ccm der Gießflüssigkeit in der angegebenen Wirkstoffkonzentration, bezogen auf 100 ccm Erde, gegossen.

Die so behandelten Pflanzen werden nach der Behandlung mit einer wäßrigen Sporensuspension von Phytophthora infestands inokuliert. Die Pflanzen werden in eine Feuchtekammer mit einer Luftfeuchtigkeit von 100 % und einer Temperatur von 18 bis 20°C gebracht. Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Eine deutliche Überlegenheit in der Wirksamkeit gegenüber dem Stande der Technik zeigen in diesem Test z.B. die Verbindungen gemäß folgender Herstellungsbeispiele: 2, 1, 4, 5, 7, 9 und 8.

## Patentansprüche

1. Pyrazolsubstituierte Oximino-cyan-acetamid-Derivate der allgemeinen Formel

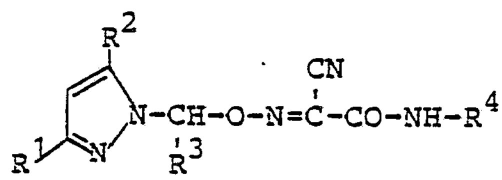

(I)

in welcher

$R^1$ und $R^2$ für Wasserstoff und Alkyl mit bis zu 3 Kohlenstoffatomen stehen,

$R^3$ für Wasserstoff, Alkyl mit bis zu 6 Kohlenstoffatomen und Halogenalkyl mit bis zu 2 Kohlenstoffatomen und bis zu 5 Halogenatomen steht,

$R^4$ für Wasserstoff und die Gruppe $CO-NH-R^5$ steht, wobei

$R^5$ für Wasserstoff und Alkyl mit bis zu 8 Kohlenstoffatomen steht, wobei letzteres durch Cyano-, Hydroxycarbonyl-, Aminocarbonyl- und Alkoxycarbonyl-Gruppen mit bis zu 4 Kohlenstoffatomen im Alkylteil substituiert sein kann.

2. Pyrazolsubstituierte Oximino-cyan-acetamid-Derivate der allgemeinen Formel (I) in Anspruch 1, wobei

$R^1$ und $R^2$ für Wasserstoff stehen,

$R^3$ für Wasserstoff, Methyl, Ethyl, Isopropyl und sek.-Butyl steht,

$R^4$ für Wasserstoff und die Gruppe $CO-NH-R^5$ steht, und

$R^5$ für Wasserstoff, Methyl, Ethyl, n-Propyl und Isopropyl steht.

3. Verfahren zur Herstellung von pyrazolsubstituierten Oximino-cyan-acetamid-Derivaten der allgemeinen Formel

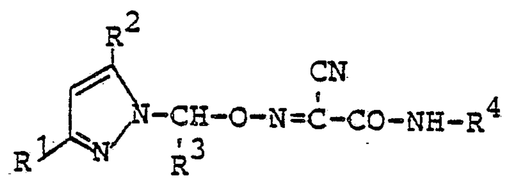

(I)

in welcher

$R^1$ und $R^2$ für Wasserstoff und Alkyl mit bis zu 3 Kohlenstoffatomen stehen,

$R^3$ für Wasserstoff, Alkyl mit bis zu 6 Kohlenstoffatomen und Halogenalkyl mit bis zu 2 Kohlenstoffatomen und bis zu 5 Halogenatomen steht,

$R^4$ für Wasserstoff und die Gruppe $CO-NH-R^5$ steht, wobei

$R^5$ für Wasserstoff und Alkyl mit bis zu 8 Kohlenstoffatomen steht, wobei letzteres durch Cyano-, Hydroxycarbonyl-, Aminocarbonyl- und Alkoxycarbonyl-Gruppen mit bis zu 4 Kohlenstoffatomen im Alkylteil substituiert sein kann,

dadurch gekennzeichnet, daß man

a) ein N-Alkylpyrazol der Formel

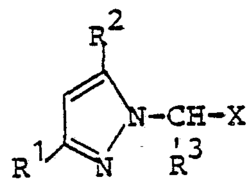

(II)

in welcher

$R^1$, $R^2$ und $R^3$ die oben angegebene Bedeutung besitzen, und

X für eine Abgangsgruppe wie Chlor, Brom, Jod oder eine Sulfonyloxygruppe steht,

mit einem 2-Oximino-2-cyan-acetamid-Derivat der Formel

$$NO-N=\overset{CN}{\overset{|}{C}}-CO-NH-R^4 \qquad (III)$$

in welcher

$R^4$ die obengenannte Bedeutung besitzt,

umsetzt; ferner daß man

b) diejenigen pyrazolsubstituierten Oximino-cyan-acetamid-Derivate der Formel (I), bei denen $R^4$ für Wasserstoff und die Gruppe $CO-NH_2$ steht, auch dann erhält, wenn man ein N-Alkyl-pyrazol der Formel (II) mit einem Alkali- oder Erdalkalisalz von 2-Oximino-2-cyan-acetamid bzw. von N-(2-Oximino-2-cyan-acetyl)-harnstoff umsetzt; ferner daß man

c) diejenigen pyrazolsubstituierten Oximino-cyan-acetamid-Derivate der Formel (I), bei denen $R^4$ für die Gruppe $CO-NH-R^5$ steht, wobei $R^5$ für die obengenannten Bedeutungen unter Ausschluß von Wasserstoff und Hydroxycarbonylalkyl steht, auch dann erhält, wenn man gemäß den Verfahren a) oder b) hergestellte erfindungsgemäße Verbindung der Formel

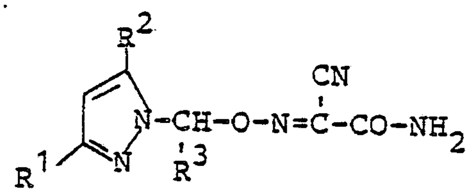

(IV)

in welcher

$R^1$, $R^2$ und $R^3$ die obengenannten Bedeutungen besitzen,

in Gegenwart einer starken Base mit einem Isocyanat der Formel

$$OCN-R^5 \qquad (V)$$

in welcher

$R^5$ die obengenannten Bedeutungen mit Ausnahme von Wasserstoff und Hydroxycarbonylalkyl besitzt,

umsetzt; und schließlich gemäß einer weiteren Verfahrensvariante

d) zur Herstellung solcher Verbindungen der Formel (I), bei denen $R^4$ für $CO-NH-R^5$ steht und $R^5$ dabei die Bedeutung Alkyl-carbonyloxyalkyl annimmt, eine nach Verfahren a) synthetisierte Carbonsäure der Formel

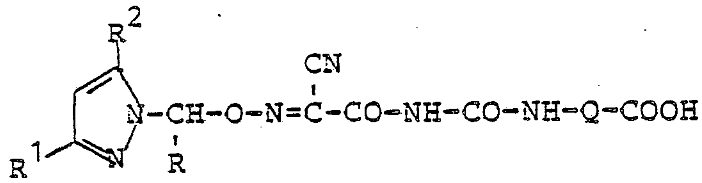

(VI)

in welcher

$R^1$, $R^2$ und $R^3$ die obengenannten Bedeutungen besitzen und

Q für gerades oder verzweigtes Alkylen mit 1 bis 5 Kohlenstoffatomen steht,

in Form ihres Alkali-, Erdalkali- oder Ammoniumsalzes mit einem Alkylierungsmittel der Formel

$$Y-R^6 \qquad (VII)$$

in welcher

$R^6$ für Alkyl mit 1 bis 3 Kohlenstoffatomen steht und

Y für eine Abgangsgruppe wie Chlor, Brom, Jod, Alkoxysulfonyloxy, Alkylsulfonyloxy oder Arylsulfonyloxy steht,

umsetzt.

4. Fungizide Mittel, gekennzeichnet durch einen Gehalt an mindestens einem pyrazolsubstituierten Oximinocyano-acetamid-Derivat der Formel (I) in Ansprüchen 1 und 3.

5. Verfahren zur Bekämpfung von Pilzen, dadurch gekennzeichnet, daß man pyrazolsubstituierte Oximino-cyan-acetamid-Derivate der Formel (I) in Ansprüchen 1 und 3 auf Pilze oder ihren Lebensraum einwirken läßt.

6. Verwendung von pyrazolsubstituierten Oximino-cyan-acetamid-Derivaten der Formel (I) in Ansprüchen 1 und 3 zur Bekämpfung von Pilzen.

7. Verfahren zur Herstellung von fungiziden Mitteln, dadurch gekennzeichnet, daß man pyrazolsubstituierte Oximino-cyan-acetamid-Derivate der Formel (I) in Ansprüchen 1 und 3 mit Streckmitteln und/oder oberflächenaktiven Mitteln vermischt.

0051784

Europäisches Patentamt

# EUROPÄISCHER RECHERCHENBERICHT

Nummer der Anmeldung

EP 81 10 8890

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D | DE - A - 2 312 956 (E.I. DU PONT DE NEMOURS)<br>* Seiten 1-4 * | 1 | C 07 D 231/12<br>A 01 N 43/56 |
| | -- | | |
| D | US - A - 3 957 847 (S.H. DAVIDSON)<br>* Spalten 1-3 * | 1 | |
| | -- | | |
| D | EP - A - 0 000 023 (BAYER AG)<br>* Seiten 34-35 * | 1 | RECHERCHIERTE SACHGEBIETE (Int. Cl.³) |
| | -- | | |
| D | US - A - 3 919 284 (K. LIN)<br>* Spalten 1-3 * | 1 | C 07 D 231/12<br>A 01 N 43/56 |
| | ---- | | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 01-02-1982 | BRIGHENTI |

EPA form 1503.1 06.78